# EUROPEAN PATENT APPLICATION

(11) **EP 4 737 011 A2**
(43) Date of publication of application: **06.05.2026**
(21) Application number: 26164944.6
(22) Date of filing: 13.04.2022
(51) Int. Cl.: B03C 3/04

(54) **CABIN AIR FILTER SYSTEM FOR A VEHICLE AND METHOD FOR CLEANING AIR TO BE SUPPLIED TO A CABIN**

(62) Divisional of application: 22305534.4
(71) Applicant: MANN+HUMMEL GmbH, 71636 Ludwigsburg (DE)
(72) Inventor: MÜNKEL, Karlheinz, 75038 Oberderdingen-Flehingen (DE); NARDINI, David, 96103 Hallstadt (DE); SCHÖN, Mirco, 95197 Schauenstein (DE); WOITOLL, Ina, 74360 Ilsfeld (DE); SIEGELE, Thomas, 71727 Benningen (DE); ZERILLI, Matthew, Kalamazoo, MI, 49004 (US); KUNZE, Stefan, 72076 Tübingen (DE); LARDEUX, Sebastien, 53410 Saint Ouen des Toits (FR); KRAUTNER, Christoph, 84163 Marklkofen (DE); BAUCH, Maximilian, 74357 Bönnigheim (DE)
(74) Representative: Mann + Hummel Intellectual Property

(57) **Abstract**

The present invention relates to a cabin air filter system (100) for a vehicle, comprising at least one particle filter element (5) and a cyclone separator (2) positioned upstream of the at least one particle filter element (5). The cabin air filter system (100) comprises a particle agglomeration device (1) positioned upstream of the cyclone separator (2). According to a second aspect the present invention relates to a method for cleaning air to be supplied to a cabin, in particular a cabin of a vehicle, with a cabin air filter system.

## Description

### Technical field

The present invention relates to a cabin air filter system for a vehicle and a method for cleaning air to be supplied to a cabin, in particular a cabin of a vehicle, in particular of a motor vehicle.

### Prior art

Vehicular cabin air filter systems have been known for a long time. Those systems typically include at least one particle filter element that comprises a porous particle filtration media adapted to remove at least a portion of the particles contained in a raw air flow. The raw air flow can either originate from the outside environment or from the cabin itself, wherein the latter first of all applies to a recirculation mode of the cabin air filter system.

Typical particle filter elements are to be replaced as soon as their dust holding capacity is reached which is usually done in predefined service intervals, e.g. annually or after a certain number of driven kilometers. Over the lifetime of a vehicle the regular replacement of cabin air filter elements can be considered a significant maintenance effort and a sustainability issue as the used filter elements are typically scrapped, e.g. by thermal processing.

In the prior art there are approaches to prolong the lifetime of cabin air filter elements by applying pre-separation with wear-free separation devices so that only a fraction of a totality of particles contained in the raw air reaches the particle filter element. Some instances of such systems are known from WO 2006/099852 A1 and WO 2005/118149 A1 for example that combine a conventional particle filter element with a cyclone pre-separator positioned upstream of the particle filter element. Although the implementation of a cyclone pre-separator has a certain impact on the lifetime of the particle filter element it cannot be considered sufficient.

The downside of such systems is that cyclone separators have an efficiency that strongly depends on the particle diameter. While cyclones can exhibit relatively high separation rates in the range of 70% or even more with particles having a diameter above approximately 5µm their separation efficiency is rather poor with smaller particles.

This is to say that the approaches disclosed in the prior art may be a functional solution for the fraction of larger particles however do not provide a significant pre-separation for the fraction of smaller particles. In the systems described in the prior art these smaller particles with a diameter below approximately 5µm still have to be almost completely separated by the particle filter element.

This especially is especially challenging as both end customers and vehicle OEMs are having increasing interest in cabin air quality and a general industrial trend towards HEPA-filtration can be observed. The smallest fraction of particles contained in the ambient air that is considered to be most detrimental to the human health and that contributes with a major share to the loading of fine particle filtration elements, especially of HEPA elements, practically is not removable at all by the cyclones of the systems described in the prior art.

So, simply combining a HEPA filter element with a cyclone pre-separator would not lead to a significant pre-separation in the smallest particle fraction and thus not lead to an increased lifetime of the HEPA filter element.

### Summary of the invention

In the view of the above it is therefore an object of the invention to provide a cabin air filter system that has a significantly increased service life of a particle filter element used therein.

This object is solved by a cabin air filter system with the features of independent claim 1.

According to a first aspect of the invention the cabin air filter system for a vehicle comprises at least one particle filter element and a cyclone separator positioned upstream of the at least one particle filter element. The cabin air filter system further comprises a particle agglomeration device positioned upstream of the cyclone separator.

The effect of the particle agglomeration device is that it increases an effective diameter of at least a portion of a totality of particles contained in a raw air. In other words, the particle agglomeration device effects a displacement of the particle spectrum to larger particle sizes. This has the advantage that the fraction of larger particles that can be removed with the cyclone separator is increased and by that the amount of particles that has to be removed with the particle filter element is minimized. This results in a significantly increased service life of the filter element. In some instances of the invention the service life of the particle filter element can be increased in a way that replacement is only necessary after 7 or even more years or after several 100.000 kilometers driven.

The cyclone separator can comprise at least one cyclone cell, in particular an axial cyclone cell. In embodiments the cyclone separator can comprise a multitude of axial cyclone cells arranged together in an axial multi cyclone cell array. The cyclone separator can comprise a dust discharge that is adapted to release the particles captured by the latter. However, the invention is not limited to cyclones and can comprise other types of inertial separators as well, e.g. baffle plates, elbow separators, that shall fall under the definition of a cyclone separator too. In embodiments the dust discharge of the cyclone separator can be coupled to a dust collection device so that the particles captured by the cyclone separator are retained and not released back to the environment. In some instances of the invention the dust discharge can be connected to a vacuum source in order to increase the efficiency of the pre-separation.

In some instances of the invention the cyclone separator can comprise a selectively switchable array of axial cyclone cells. In that the cyclone separator can be provided with at least one switching element that is adapted to selectively activate or de-activate a predefined number of cyclone cells. As the efficiency of cyclone separators strongly depends from the volume flow this has the potential to operate the cyclone cells with individual volume flows close to their optimum efficiency point under a broader range of operating conditions of the cabin air filter system.

In embodiments the particle filter element can comprise at least one layer of a porous particle filtration media. The particle filtration media especially being provided in the form of a filtration medium body, in that the particle filtration media is in particular pleated to form a filtration media bellows. The filtration medium body can have any shape and/or size. In particular the filtration medium body can have a cuboid shape or be provided in the form of a hollow cylinder. In other embodiments the filtration medium body can have the shape of a hollow cone or be provided in a tube shape. "Round" filtration medium body types (hollow cylindrical, hollow conical, tube) can be especially flown through by the air to be purified in a radial direction, in particular from a radially inner side to a radially outer side.

In principle the invention comprises any agglomeration device that is functionable to efficiently increase the effective diameter of at least a fraction of particles contained in the raw air.

In an embodiment the particle agglomeration device comprises at least one of: A mono-polar ionizer, a bi-polar ionizer, an electrostatic discharge device, a sound source, in particular ultrasound source, a humidification device, a magnetic-effect device.

In another embodiment the cabin air filter system comprises a bypass duct around the at least one particle filter element and a flap that is adapted to switch the bypass duct. In a filtration mode an air flow is processed by the at least one particle filter element and in a bypass mode an air flow is bypassed through the bypass duct and does not or only partially pass through the particle filter element. This has the further advantage that the particle filter element is only "used" when required which further extends the service life of the particle filter element. The bypass can be activated when the raw air quality is clean enough to fulfil predefined specifications so that a subsequent particle filter treatment is unnecessary after the air has been processed by the cyclone separator. This may for example be the case when a vehicle equipped with the cabin air filter system according to the invention is operated in clean rural areas or in environments that are exclusively polluted with coarse dust that can be sufficiently removed by the cyclone.

In a preferred embodiment the particle filter element is a HEPA filter element, in particular a HEPA filter element that fulfils specification H13 according to EN 1822 or better. The filtration media used in the HEPA filter element may comprise at least one fine filtration layer comprising glass fibers, synthetic nanofibers and/or a membrane, in particular comprising expanded PTFE. The filtration media may comprise additional layers that can act as a support or pre-filtration layer for the fine filter layer. An additional layer may comprise a synthetic nonwoven that can comprise various plastic materials such as PET, PP, PC and the like.

In another embodiment the cabin air filter system comprises an airstream generation device, in particular a blower, wherein the blower is in particular positioned downstream of the particle filter element. This is to say the particle filter element may be arranged on a suction side of the blower. In other instances the particle filter element may be however equally arranged on the pressure side of the blower. The blower can be selected from the group comprising: An axial blower, a radial blower, a diagonal blower.

In other instances of the invention the cabin air filter system can comprise at least one filter housing that provides a receiving space in that the at least one particle filter is received. The filter housing provides in particular can provide a sealing surface on that the at least one particle filter element sealingly abuts so as to tightly seal an upstream raw side from a downstream clean side of the particle filter element.

Other components of the system may be arranged in the filter housing as well. However it is possible that other components of the system have own housings. The components of the system can be fluidically connected to a neighboring component by at least one air duct each.

In yet another embodiment the cabin air filter system comprises an adsorption filter element that is spatially separated from the particle filter element, wherein the adsorption filter element is in particular positioned downstream of the airstream generation device.

The adsorption filter element can be in particular a regenerable adsorption filter element so that its lifetime is practically unlimited. This is feasible as most adsorption processes are at least partially reversible. The technical advantage correlating to this is that the system function "gas adsorption" is separated from the particle filtration so that at least for the gas adsorption a truly increased service life can be provided by the system according to the invention. The adsorption filter can be adapted to remove harmful gases from an air flow to be processed; typical harmful respectively unwanted gases include NOₓ, CO, SO₂, VOCs (volatile organic compounds) and potentially even CO₂. Additionally the adsorption filter may be adapted to remove smells.

In a further embodiment the cabin air filter system comprises an outside air inlet that is adapted to receive outside air, wherein the outside air inlet is positioned upstream of the particle agglomeration device. Alternatively or additionally the cabin air filter system comprises a cabin air outlet that is adapted to feed an air flow processed by the cabin air filter system to a cabin, wherein the cabin air outlet is in particular positioned downstream of the adsorption filter element. In some instances of the invention the cabin air outlet can be directly connected to an air volume of the cabin whereas in other embodiments the cabin air outlet may be connected to a respective air inlet of a subsidiary air treatment device like an air-conditioning system. The outside air inlet may be provided in fluid communication with a suitable air intake device that can be provided on the vehicle in a variety of positions, e.g. at a firewall below the windscreen.

Beyond that the cabin air filter system in embodiments may comprise an alternative inlet, in particular a recirculation inlet, that is adapted to receive air from the cabin, wherein the alternative inlet is in particular positioned downstream of the cyclone separator and upstream of the at least one particle filter element. The alternative inlet can be used in an implementation of the cabin air filter system according to the invention to realize a recirculation mode in that an air flow to be processed is taken from the cabin and not from the environment. The use of a recirculation mode has various advantages that relate to potential energy savings on the one hand (recirculated air from the cabin is already thermally pre-conditioned) and has the potential to improve the particle filter service life (especially under high outside pollution levels as the recirculated air from the cabin is already precleaned). With the cabin air filter system according to the invention a recirculation air flow can be both treated with particle filtration and gas adsorption independently. If the cabin air filter system according to the invention is used in an implementation together with a subsequently arranged air-conditioning device there is no need to provide a recirculation filter in the air-conditioning device which is beneficial as system complexity can be reduced.

In yet another embodiment the cabin air filter system comprises an inlet selection flap that is adapted to selectively switch the alternative inlet. The inlet selection flap can have at least two switching positions: In a first switching position an outside air inlet is active so that an air flow can be drawn from the cyclone separator, in a second switching position the cabin air inlet is active so that an air flow can be drawn from the cabin. The inlet selection flap may in particular be operated by an actuator, especially an electro-mechanical actuator.

According to a further embodiment the cabin air filter system comprises at least one heating element adapted for heating the adsorption filter for regeneration, wherein the heating element comprises at least one of: A resistance heater, a PTC heater, a heat exchanger for a heat transfer medium. The temperature that is required to effect a regeneration of the adsorption filter element depends on the material composition, structure and dimensions of the adsorption filter. Typical regeneration temperatures may range from 70 to 150°C.

In another embodiment the heating element may comprise at least one heating means that is adapted to use heat from a battery system. In particular the heating means may be adapted to use an excess heat resulting from a charging and/or discharging process of a vehicular battery system, in particular a traction battery system. This has the advantage that this amount of heat does not affect a driving range.

In another embodiment the at least one heating element may additionally be used for heating purposes independently from the regeneration function. This may be especially beneficial under cold-start conditions and/or to allow for a quick defrosting or defogging of windows of a vehicle equipped with the cabin air filter system according to the invention.

The heating element may be thermally conductively coupled to the adsorption filter or can be positioned upstream of the adsorption filter so as to heat a regeneration air flow to be processed by the adsorption filter.

In another embodiment the cabin air filter system comprises a regeneration flap, wherein the regeneration flap is adapted to switch an adsorption outlet to the environment. The regeneration flap can be in particular positioned downstream of the adsorption filter. The adsorption outlet is adapted to release the harmful gases that are released from the adsorption filter element during regeneration to the environment.

The regeneration flap may in particular be operated by an actuator, especially an electromechanical actuator.

The cabin air filter system can be shiftable into a regeneration mode in that the adsorption filter is regenerated .

In the regeneration mode:
- the heating element is activated so that the adsorption filter element is heatable to a temperature above a predefined regeneration temperature and
- the regeneration outlet is opened by the regeneration flap so that the regeneration air flow processed by the adsorption filter is releasable to the environment.

It is in particular possible that the regeneration mode is activated when a vehicle equipped with said cabin air filter system is parked and in a particular while the vehicle is charged. This has the advantage that the vehicle's energy storage is not stressed by an energy demand required to effect regeneration respectively desorption of the adsorption filter element.

In a preferred embodiment the adsorption filter element comprises at least one adsorbent honeycomb, in particular an activated carbon honeycomb. Adsorbent honeycombs have the main advantage over other adsorbent bodies like pourings of adsorbent particles or wound or stacked layers of adsorbent filled media that they provide a drastically lower resistance to the flow and thus create significantly lower pressure loss. The adsorbent honeycombs can have any cross section as they are typically produced by extrusion, for instance they can have a rectangular or circular cross section. It is in particular possible that the adsorption filter comprises more than one adsorbent honeycombs, for example four, wherein the individual adsorbent honeycombs can be arranged in the same plane neighboring each other. Contact regions between neighboring honeycombs are preferably sealed in an appropriate manner so as to avoid a bypass flow inbetween.

In an exemplary embodiment the dimensions of an individual adsorbent honeycomb can be:
- Length: 30-70 mm, in particular 35-65 mm, in particular approximately 40 mm
- Width: 35-75 mm, in particular 40-65mm, in particular approximately 50 mm
- Height: 35-75 mm, in particular 40-65mm, in particular approximately 50 mm

The "length" defining an extension in axial direction of the channels provided in the adsorbent honeycomb. "Width" and "Height" defining an extension of the adsorbent honeycomb in a plane oriented normally to the axial direction.

The adsorbent honeycombs can have a channel density of 200-600 channels per square inch (~31-93 channels per square centimeter), in particular 300-500 channels per square inch (~46,5-77,5 channels per square centimeter), in particular approximately 400 channels per square inch (~62 channels per square centimeter).

In order to provide an effective sealing interface against an adsorption filter housing the at least one honeycomb can be connected to a circumferential frame that provides a circumferential seal collar that can carry a seal.

The activated carbon honeycomb may comprise one or multiple types of activated carbon that are each tailored to a specific target gas spectrum. The activated carbon honeycomb may additionally or alternatively comprise at least one impregnation that is adapted to address even further target gases.

According to another embodiment the cabin air filter system can comprise at least one photocatalytic oxidation (PCO) device. The PCO device can comprise at least one air permeable carrier that comprises a photocatalytic active material, e.g. a metal oxide like TiO₂. The active material can be in particular coated onto the air permeable carrier but also be introduced thereto in any other technically feasible way. At an opposing side of the air permeable carrier an UV-A light source is positioned so that at least a portion of the air permeable carrier that is equipped with the active material can be illuminated for activation of the active material. By PCO harmful substances like VOC, CH₂O (Formaldehyde), Viruses, Bacteria can be removed or at least be reduced.

The air permeable carrier of the PCO device can be embodied as a separate carrier structure like a grid , mesh and/or nonwoven. Alternatively the air permeable carrier can be at least a section of the particle filter element, in particular of the HEPA filter element.

The PCO device can be arranged anywhere in the cabin air filter system, for example
- downstream of the particle filter element and upstream of the blower and/or
- upstream of the adsorbent honeycomb and downstream of the blower and/or
- downstream of the adsorbent honeycomb.

A combination of an adsorbent honeycomb and PCO device is particularly advantageous as the individual target gas spectra complement each other.

In another embodiment the carrier that contains the active material can be impermeable to air. In particular at least a portion of at least one housing component and/or at least a portion of at least one duct of the cabin air filter system can be coated with the active material. Also this embodiment requires at least one UV-A light source being arranged opposite to the coated surface portion so that the active material can be illuminated for activation.

Furthermore the cabin air filter system can additionally or alternatively comprise at least one UV treatment device comprising at least one UV-C light source. The UV-C light source is in particular adapted to treat viruses, bacteria and other germs by high energetic radiation that damages their DNA. The UV-C light source can be arranged anywhere in the cabin air filter system, especially on a downstream side of the particle filter element so that a downstream surface of the particle filter element can be illuminated with the at least one UV-C light source. This has the advantage that any microbial growth on a filtration media of the particle filter element can be avoided or at least be reduced.

In conjunction with "round" particle filter elements the UV-A light source of the PCO device and/or the UV-C light source of the UV treatment device may in particular be arranged in an inner hollow space provided by a filtration medium body of the particle filter element.

Finally, the cabin air filter system can comprise at least one nebulizer that is adapted to spray concentrated NaCl respectively a saline solution of NaCl in water to an air flow. The nebulizer can be positioned downstream of the adsorption filter, especially as a final treatment stage of the cabin air filter system. The nebulizer can be selectively activable to provide a salty atmosphere in the cabin. In other words the cabin air filter system according to this embodiment is able to provide a "sea climate mode" when the nebulizer is activated.

Lastly the cabin air filter system in an additional embodiment might be equipped with at least one oxygen tank that contains compressed oxygen or a mixture of oxygen with other gases. The oxygen tank can be fluidically connected to at least one nozzle provided in a duct or housing component of the cabin air filter system. The fluidic connection between the oxygen tank and the nozzle can be selectively activable by a valve. When the valve is opened a flow of oxygen or a mixture of oxygen with other gases from the tank to the nozzle is effected that results in an increased oxygen concentration in the cabin. In other words, the cabin air filter system according to this embodiment is able to provide a "oxygen boost mode" when the valve is opened. This may be especially useful to arrange for an overpressure in the cabin that can be required under certain operating conditions of a vehicle equipped with the cabin air filter system according to the invention.

According to another aspect of the invention a method for cleaning air to be supplied to a cabin, in particular a cabin of a vehicle, is proposed. The cabin air filter system comprises at least one particle filter element, a cyclone separator positioned upstream of the at least one particle filter element and a particle agglomeration device positioned upstream of the cyclone separator.

The method comprising the steps:
- processing a raw air flow received from the outside in the particle agglomeration device, thereby increasing an effective diameter of at least a portion of a totality of particles contained in the raw air flow,
- processing an air flow processed by the particle agglomeration device in the cyclone separator, thereby by inertial action removing at least a portion of a totality of particles contained in the air flow processed by the particle agglomeration device in the cyclone separator,
- processing an air flow processed by the cyclone separator in the particle filter element, thereby removing at least a portion of a totality of particles contained in the air flow processed by the cyclone separator in the particle filter element,
- supplying the air flow processed by the particle filter element to the cabin.

The step of "supplying the air flow processed by the particle filter element to the cabin" can include a direct supply of said air flow to the cabin or an indirect supply. Indirect can in particular have the meaning that only a partial flow of the air flow processed by the particle filter element is supplied to the cabin. Alternatively or additionally indirect can also have the meaning that subsequent cleaning steps of the air flow processed by the particle filter element are performed.

Any features, combination of features and their specific technical advantages that have been described herein for the cabin air filter system according to the invention are applicable to the method according to the invention as well and vice versa.

### Brief description of the drawings

Further advantages can be understood from following description of the drawings. The drawings show examples of embodiments of the invention. The drawings, the description and the claims contain numerous features in combination. The skilled person will expediently also consider the features individually and combine them to form useful further combinations. The drawings show exemplary:
- **Fig. 1**: system chart of the cabin air filter system according to a first embodiment;
- **Fig. 2**: system chart of the cabin air filter system according to a second embodiment;
- **Fig. 3**: system chart of the cabin air filter system according to a third embodiment;
- **Fig. 4**: system chart of the cabin air filter system according to a fourth embodiment;
- **Fig. 5**: system chart of the cabin air filter system according to a fifth embodiment;
- **Fig. 6**: system chart of the cabin air filter system according to a sixth embodiment.

### Embodiments of the invention

In the drawings, the same or similar components may be marked with the same reference signs. The figures only show examples and are not to be understood as limiting.

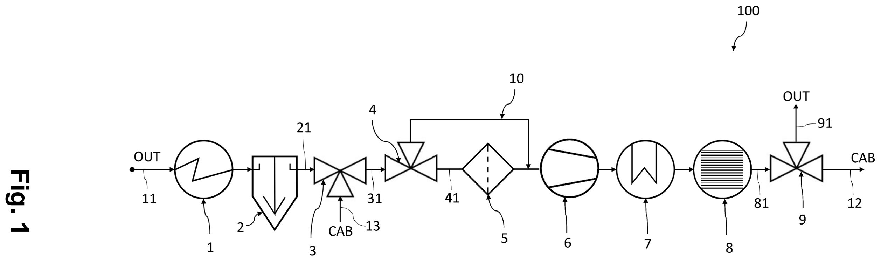

**Fig. 1** shows a system chart of a first embodiment of the cabin air filter system 100 according to the invention.

The system 100 includes an outside air inlet 11 that is adapted to draw ambient air to the system. The outside air inlet 11 may be provided in fluid communication with a suitable air intake device that can be provided on a vehicle in a variety of positions, e.g. at a firewall below the windscreen or in a luggage compartment.

Air drawn through the outside air inlet 11 is subsequently processed by a particle agglomeration device 1 and after that fed to a cyclone separator 2 for further processing.

The effect of the particle agglomeration device 1 is that it increases an effective diameter of at least a portion of a totality of particles contained in the air. In other words the particle agglomeration device 1 effects a displacement of the particle spectrum to larger particle sizes. This has the advantage that the fraction of larger particles that can be removed with the cyclone separator 2 is increased and by that the amount of particles that has to be removed with a particle filter element 5 is minimized.

The technical advantage of combining a particle agglomeration device 1 with a cyclone separator 2 can be seen in that this allows to remove a relatively large portion of a totality of particles contained in the raw air with wear-free components.

In a filtration mode of the system 100 the air processed by the cyclone separator 2 is fed to particle filter element 5 that can especially be a HEPA filter element 5. The particle filter element 5 provides "classical" filtration by a porous filtration media to remove a residual amount of particles that have not been removed by the cyclone separator 2.

Downstream of the particle filter element 5 a blower 6 is positioned so that the blower is adapted to draw air through the upstream system components. In other words the particle agglomeration device 1, the cyclone separator 2 and the particle filter element 5 may be arranged on a suction side of the blower 6.

Downstream of the blower 6 an adsorption filter element 8 is positioned. The adsorption filter element is spatially separated from the particle filter element 5. The adsorption filter element 8 can be adapted to remove harmful gases from an air flow to be processed; typical harmful respectively unwanted gases include NOₓ, CO, SO₂, VOCs and potentially even CO₂. In particular the adsorption filter element 8 can comprise at least one adsorbent honeycomb, especially an activated carbon honeycomb, which is beneficial for pressure loss reasons.

The adsorption filter element 8 is regenerable so that its lifetime is practically unlimited. This is practically feasible as most adsorption processes are at least partially reversible. The technical advantage correlating to this is that the system function "gas adsorption" is separated from the particle filtration so that at least for the gas adsorption a truly increased service life can be provided by the system according to the invention.

For regeneration of the adsorption filter element 8 it has to be heated to a temperature above a predefined regeneration temperature that typically ranges around 70°C. The heating of the adsorption filter element 8 for regeneration purposes is implemented by a heating element 7 positioned upstream of the adsorption filter 8.

After the air flow has been processed by the adsorption filter element 8 in the filtration mode the air flow is supplied to the cabin through cabin outlet duct 12 of the system 100.

In order to switch the system 100 into the regeneration mode an outlet side of the adsorption filter element 8 is fluidically connected to a regeneration outlet 91 that leads to the environment. Structurally this is realized by a regeneration flap 9 with two outlets and one inlet. The regeneration flap 9 has one inlet connected to a duct 81 that is connected to the outlet side of the adsorption filter element 8. The regeneration flap has two outlets, wherein a first outlet is connected to the regeneration outlet 91 and a second outlet is connected to the cabin outlet duct 12.

Further, in the regeneration mode the heater 7 is activated to heat a regeneration air flow to be processed by the adsorption filter element 8. By the effect of heat harmful gases that have been adsorbed in the adsorption filter element 8 are released and finally fed to the environment.

The regeneration air flow can either originate from the environment (through outside inlet 11) or from the cabin (through alternative inlet 13). Using air from the cabin for regeneration purposes may be beneficial from an energetic perspective as an air volume of the cabin is at least thermally pre-conditioned.

The system 100 further includes an inlet selection flap 3 that is adapted to selectively switch the alternative inlet 13 that receives air from the cabin. The alternative inlet in other words can be a recirculation inlet. The inlet selection flap 3 is positioned downstream of the cyclone separator 2 and upstream of the at least one particle filter element 5. The inlet selection flap 3 has two inlets and one outlet.

The inlet selection flap 3 includes an outlet that is connected by a duct 31 to an inlet side of a bypass flap 4. The inlet selection flap 3 has a first inlet connected to the cabin inlet duct 13 and a second inlet connected to a duct 21 that is connected to an outlet side of the cyclone separator 2. In a filtration mode an air flow received from the cyclone separator 2 is routed to the particle filter element 5 via the bypass flap 4. In a recirculation mode an air flow received from the cabin inlet duct 13 is routed to the particle filter element 5 via the bypass flap 4. As outlined above this recirculation mode inlet configuration may be used in the regeneration mode as well.

Lastly the system 100 comprises a bypass flap 4 and a bypass duct 10 around the particle filter element 5. The bypass duct 10 branches off upstream of the particle filter element 5 and re-joins a main duct downstream of the particle filter element 5.

The particle filter element 5 thus can be bypassed under certain operating conditions in that a subsequent processing of an air flow by the particle filter element 5 is not necessary.

The bypass flap 4 includes one inlet and two outlets. The inlet is connected to the duct 31 that is connected to the outlet side of the recirculation flap 3. A first outlet of the bypass flap 4 is connected to the bypass duct 10 and a second outlet of the bypass flap 4 is connected a duct 41 being in fluidic communication with the particle filter element 5.

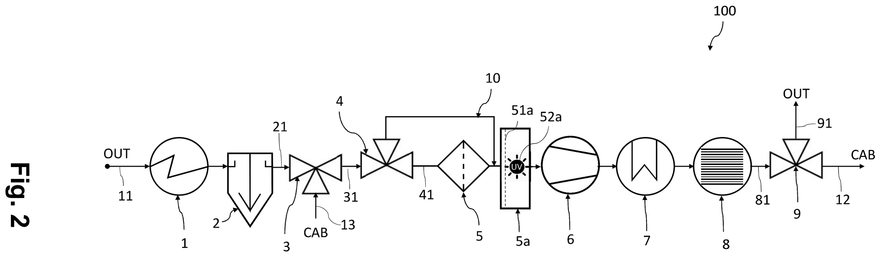

The system 100 according to the second embodiment depicted in **Fig. 2** in principle has the same system components and operates analogously. The difference is that it comprises a PCO device 5a downstream of the particle filter element 5. The PCO device 5a may be in particular arranged upstream of the blower 6.

The PCO device 5a comprises an air permeable carrier 51a that may comprise a grid, mesh and/or nonwoven that is equipped with at least one active substance that may in particular comprise TiO₂. The PCO device 5a comprises at least one UV-A light source that is arranged opposite to the air permeable carrier 51a such that the active substance of the air permeable carrier 51a can be illuminated to activate the active substance.

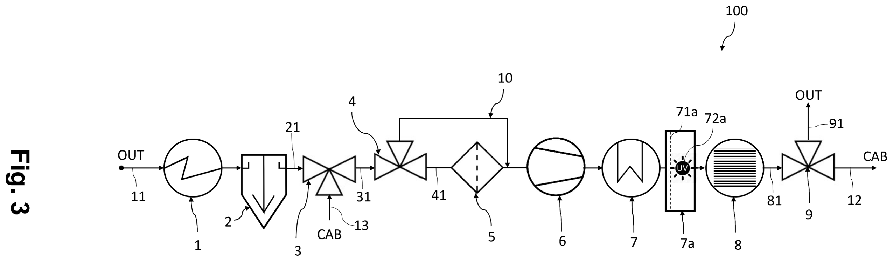

The system 100 according to the third embodiment depicted in **Fig. 3** in principle has the same system components and operates analogously. However a difference is the positioning of the PCO device 7a comprising the air permeable carrier 71a and the at least one UV-A light source 72a. The PCO device 7a is arranged upstream of the adsorption filter element 8 and downstream of the heater 7.

The positioning of the PCO device 5a,7a shown in Fig. 2 and Fig. 3 are only to be understood exemplary. The invention explicitly comprises alternatives for their positioning as well.

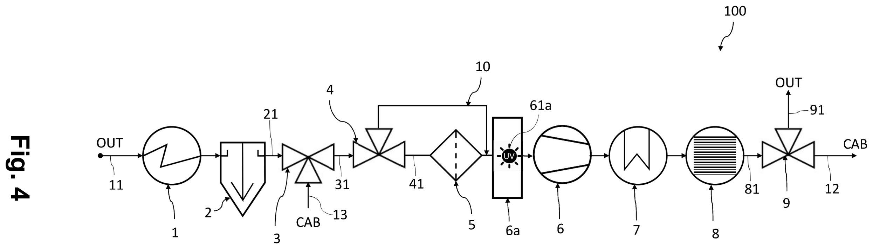

The system 100 according to the fourth embodiment depicted in **Fig. 4** in principle has the same system components and operates analogously. However there is a difference. The UV treatment device 6a positioned downstream of the particle filter element 5 and upstream of the blower 6 may comprise a UV-A or UV-C light source 61a.

In the case it comprises a UV-A light source 61a it may be used in conjunction with the particle filter element 5 as an alternative PCO device. In that regards at least a portion of the particle filter element 5 is equipped with an active substance such as TiO2 that can be illuminated with the UV-A light source 61a for activation.

In the case the UV treatment device 6a comprises a UV-C light source it may be used without an active substance on the particle filter element 5 as an additional air treatment stage that is in particular adapted to treat viruses, bacteria and other germs by high energetic radiation that damages their DNA.

An UV treatment device 6a comprising an UV-C light source may - in all embodiments - be used additionally to the PCO device 5a, 7a and can be practically positioned at any position in the system and explicitly not only in the ones depicted in the drawings.

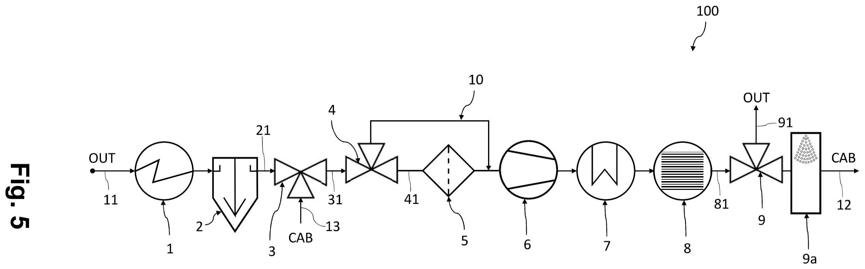

The system 100 according to the fifth embodiment depicted in **Fig.** 5 in principle has the same system components and operates analogously as the system according to the first embodiment. The system 100 includes a nebulizer 9a that is adapted to spray concentrated NaCl respectively a saline solution of NaCl in water to an air flow. The nebulizer 9a is positioned downstream of the adsorption filter element 8 as a final treatment stage of the cabin air filter system 100. The nebulizer 9a can be selectively activable to provide a salty atmosphere in the cabin. The nebulizer 9a is in particular positioned downstream of the regeneration flap 9.

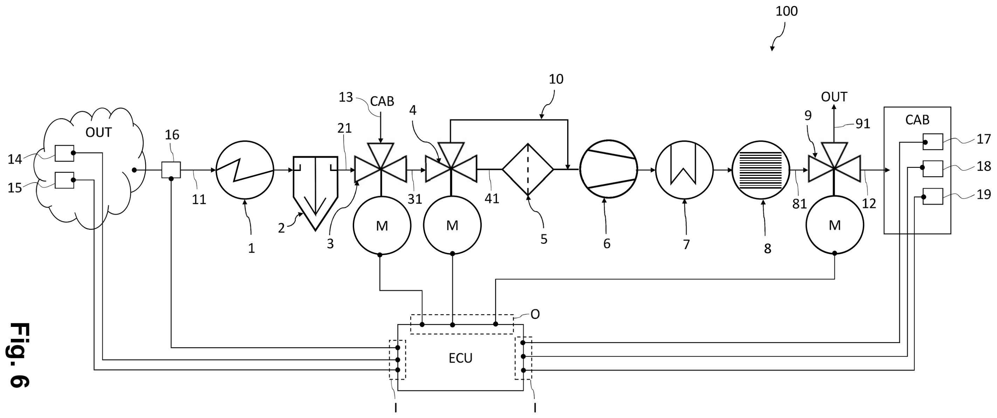

The system 100 according to the fifth embodiment depicted in **Fig.** 6 in principle has the same system components and operates analogously as the system according to the first embodiment. However the sixth embodiment includes as additional components:
outside air property sensor 14, outside air property sensor 15, inlet duct air property sensor 16, cabin air property sensors 17,18,19, an electronic control unit ECU having inputs I and outputs O and actuators M coupled to the inlet selection flap 3, the bypass flap 4 and the regeneration flap 9.

The number and types of sensors and their positioning is not to be understood such that the invention would be restricted thereto. The invention explicitly comprises embodiments that comprise only one sensor (outside, duct or cabin).

Each of the actuators M is adapted to operate one of the flaps comprising the inlet selection flap 3, the bypass flap 4 and the regeneration flap 9.

The ECU receives as inputs I sensor signals from at least one of the sensors comprising an outside air property sensor 14, an outside air property sensor 15, an inlet duct air property sensor 16, one or more cabin air property sensors 17,18,19. The ECU controls as outputs O the actuators M.

The outside air property sensor 14 and the cabin air property sensor 17 may in particular be particulate matter sensors. The outside air property sensor 15 may be in particular a gas sensor, in particular a CO₂ sensor. The cabin air property sensor 18 may be in particular gas sensor, in particular a CO₂ sensor. The cabin air property sensor 19 may be in particular gas sensor, in particular a VOC sensor.

The ECU processes the inputs I according to a predefined algorithm to control the outputs O.

Feasible control strategies implemented in the ECU include energy saving and optimization of lifetime of the particle filter element.

### Energy saving strateqy:

With the help of at least one CO₂ sensor, preferably a cabin CO₂ sensor, it is possible to optimize the recirculation ratio of the system to increase the range of the vehicle by adjusting it to a certain maximum threshold value for the cabin CO₂ concentration. This is to say CO₂ level based control aims to operate the system as much as possible with active recirculation but in a safe range for the passenger's health. By this the power consumption of a thermal system of an air-conditioning can be reduced as a smaller volume of air needs to be cooled down respectively heated up.

The information of at least one particulate matter sensor (inside or outside) can be equally beneficially used to optimize the control of the system from an energetic perspective. The objective therein is to reduce the power consumption of the system by bypassing the particle filter element (in particular HEPA filter element) when an outside air quality is within an acceptable range, thus reducing the blower's power consumption.

### Optimization of lifetime strategy:

If the particle filter element is bypassed when an outside air quality is within an acceptable range this helps to increase the lifetime of the particle filter element as a cumulative dust loading over the lifetime thereby can be significantly reduced.

Alternatively or additionally to the use of sensor signals the ECU may process data received from external data sources in order to control the cabin air filter system. This external data may in particular comprise air property information, a current geolocation, a current driving speed and/or traffic information.

### Reference numerals

- 100: cabin air filter system
- 10: bypass duct
- 1: particle agglomeration device
- 11: outside inlet duct
- 12: cabin outlet duct
- 13: alternative inlet / cabin inlet duct
- 14: outside air property sensor
- 15: outside air property sensor
- 16: inlet duct air property sensor
- 17: cabin air property sensor
- 18: cabin air property sensor
- 19: cabin air property sensor
- 2: cyclone separator
- 21: connection duct from cyclone separator to inlet selection flap
- 3: inlet selection flap
- 31: connection duct from inlet selection flap to bypass flap
- 4: bypass flap
- 41: connection duct from bypass flap to particle filter element
- 5: particle filter element / HEPA filter element
- 51: PCO device
- 51a: air permeable carrier
- 52a: UV-A light source
- 6: airstream generation device / blower
- 6a: UV treatment device
- 61a: UV light source, UV-A or UV-C
- 7: heater
- 71: PCO device
- 71a: air permeable carrier
- 72a: UV-A light source
- 8: adsorption filter element / adsorbent honeycomb
- 81: connection duct from adsorption filter element to regeneration flap
- 9: regeneration flap
- 9a: nebulizer
- 91: regeneration outlet duct
- M: actuator
- OUT: outside / environment
- CAB: cabin
- ECU: electronic control unit
- I: inputs of ECU
- O: outputs of ECU

## Claims

1. Cabin air filter system (100) for a vehicle, comprising at least one particle filter element (5) and a cyclone separator (2) positioned upstream of the at least one particle filter element (5), **characterized in that** the cabin air filter system (100) comprises a particle agglomeration device (1) positioned upstream of the cyclone separator (2).

2. Cabin air filter system (100) according to claim 1,
**characterized in that** the particle agglomeration device (1) comprises at least one of: A mono-polar ionizer, a bi-polar ionizer, an electrostatic discharge device, a sound source, in particular ultrasound source, a humidification device, a magnetic-effect device.

3. Cabin air filter system (100) according to claim 1 or 2,
**characterized in that** the particle agglomeration device (1) is adapted to effect an increase of an effective diameter of at least a portion of a totality of particles contained in a raw air flow to be processed by the particle agglomeration device (1).

4. Cabin air filter system (100) according to any of the claims 1 to 3,
**characterized in that** the cabin air filter system (100) comprises a bypass duct (10) around the at least one particle filter element (5) and a flap (4) that is adapted to switch the bypass duct (10), wherein in a filtration mode an air flow is processed by the at least one particle filter element (5) and in a bypass mode an air flow is bypassed through the bypass duct (10).

5. Cabin air filter system (100) according to any of the preceding claims,
**characterized in that** the particle filter element (5) is a HEPA filter element, in particular a HEPA filter element that fulfils specification H13 according to EN 1822 or better.

6. Cabin air filter system (100) according to any of the preceding claims,
**characterized in that** the cabin air filter system (100) comprises an airstream generation device (6), in particular a blower (6), wherein the blower (6) is in particular positioned downstream of the particle filter element (5).

7. Cabin air filter system (100) according to any of the preceding claims,
**characterized in that** the cabin air filter system (100) comprises an adsorption filter element (8) that is spatially separated from the particle filter element (5), wherein the adsorption filter element (8) is in particular positioned downstream of the airstream generation device (6).

8. Cabin air filter system (100) according to any of the preceding claims,
**characterized in that** the cabin air filter system comprises (100)
- an outside air inlet (11) that is adapted to receive outside air, wherein the outside air inlet (11) is positioned upstream of the particle agglomeration device (1) and/or
- a cabin air outlet (12) that is adapted to feed an air flow processed by the cabin air filter system (100) to a cabin, wherein the cabin air outlet (12) is in particular positioned downstream of the adsorption filter element (8).

9. Cabin air filter system (100) according to any of the preceding claims,
**characterized in that** the cabin air filter system (100) comprises an alternative inlet (13), in particular a recirculation inlet, that is adapted to receive air from the cabin, wherein the alternative inlet (13) is in particular positioned downstream of the cyclone separator (2) and upstream of the at least one particle filter element (5).

10. Cabin air filter system (100) according to claim 9,
**characterized in that**
the cabin air filter system (100) comprises an inlet selection flap (3) that is adapted to selectively switch the alternative inlet (13).

11. Cabin air filter system (100) according to any of the claims 7 to 10,
**characterized in that** the cabin air filter system (100) comprises at least one heating element (7) adapted for heating the adsorption filter element (8) for regeneration, wherein the heating element (7) comprises at least one of: A resistance heater, a PTC heater, a heat exchanger for a heat transfer medium.

12. Cabin air filter system (100) according to claim 11,
**characterized in that** the heating element (7) is thermally conductively coupled to the adsorption filter element (8) or positioned upstream of the adsorption filter element (8) so as to heat a regeneration air flow to be processed by the adsorption filter element (8).

13. Cabin air filter system (100) according to claim 11 or 12,
**characterized in that** the cabin air filter system (100) comprises a regeneration flap (9), wherein the regeneration flap (9) is adapted to switch a regeneration outlet (91) to the environment, wherein the regeneration flap (9) is in particular positioned downstream of the adsorption filter element (8).

14. Cabin air filter system (100) according to claim 11 or 13,
**characterized in that** the cabin air filter system (100) is shiftable into a regeneration mode **in that** the adsorption filter element (8) is regenerated, wherein in the regeneration mode:
- the heating element (7) is activated so that the adsorption filter element (8) is heatable to a temperature above a predefined regeneration temperature and
- the regeneration outlet (91) is opened by the regeneration flap (9) so that the regeneration air flow processed by the adsorption filter element (8) is releasable to the environment.

15. Method for cleaning air to be supplied to a cabin, in particular a cabin of a vehicle, with a cabin air filter system, in particular a cabin air filter system (100) according to any of the preceding claims,
wherein the cabin air filter system (100) comprises at least one particle filter element (5), a cyclone separator (2) positioned upstream of the at least one particle filter element (5) and a particle agglomeration device (1) positioned upstream of the cyclone separator (2),
the method comprising the steps:
- processing a raw air flow received from the outside in the particle agglomeration device (1), thereby increasing an effective diameter of at least a portion of a totality of particles contained in the raw air flow,
- processing an air flow processed by the particle agglomeration device (1) in the cyclone separator (2), thereby by inertial action removing at least a portion of a totality of particles contained in the air flow processed by the particle agglomeration device (1) in the cyclone separator (2),
- processing an air flow processed by the cyclone separator (2) in the particle filter element (5), thereby removing at least a portion of a totality of particles contained in the air flow processed by the cyclone separator (2) in the particle filter element (5),
- supplying the air flow processed by the particle filter element (5) to the cabin.
